# EUROPEAN PATENT APPLICATION

(11) **EP 1 795 892 A1**
(43) Date of publication of application: **13.06.2007**
(21) Application number: 05801339.2
(22) Date of filing: 30.09.2005
(51) Int. Cl.: G01N 33/28, F16N 29/00, G01N 15/06

(54) **DETECTOR FOR THE PREDICTIVE DIAGNOSIS OF FAULTS IN MEDIUM- AND HIGH-POWER GEAR BOXES AND REDUCERS**

(30) Priority: 01.10.2004 ES 20042344
(71) Applicant: Metro de Madrid, S.A., 28007 Madrid (ES)
(72) Inventor: GONZALEZ FERNANDEZ, Francisco, Javier, E-28007 Madrid (ES); ALAMINOS NAVARRO, Jacinto, E-28007 Madrid (ES)
(74) Representative: Carpintero Lopez, Francisco
(86) International application number: PCT/ES2005/000529
(87) International publication number: WO 2006/037830

(57) **Abstract**

The detector is made up of an enveloping electrode or hollow plug (1) in the interior of which, coupled concentrically to it, there is a second electrode (9) formed of a bar magnet or magnetized rod with the function of attracting the ferromagnetic particles. When the number of particles grows to the extent that the electrodes come into contact with each other, the electrical circuit indicating the possible fault in the device monitored is closed.

The body of the electrode or plug (1) is divided into areas, an anterior one (2) through which the inner bar magnet electrode (9) is visible and which is immersed in the lubricating fluid to be monitored, an intermediate threaded area (3) for connection to the machine reservoir, and a third area (4) for issuance of the electrode contacts which includes means for tightening the body of the plug to the reservoir containing the lubricating fluid

## Description

### OBJECT OF THE INVENTION

The object of the present invention consists of a detector for the predictive diagnosis of faults in medium and high power gear motors and transmission cases as may be of application for instance in transmission cases of railway vehicles, railway, windmill and other types of gear motors, torque multipliers, etc.

This detector incorporates undoubted benefits compared with the present devices, as it combines not only the predictive action of knowing when there is a high degree of wear on these mechanical components, but also preventive action through attracting towards that component the particles stemming from the wear, which are eliminated from the recirculation towards the mechanical components of gear motors and transmission cases, thereby reducing wear and increasing their useful life.

This detector combines a magnetized core which attracts the ferromagnetic particles towards it, while being converted at the same time into an electrical conductor pole formed by the switch which, connected to the electrical circuit, will indicate when the wear on the mechanical components to be monitored is excessive and it is advisable for them to be repaired.

### BACKGROUND OF THE INVENTION

There are mechanical components, as may be the case of transmission cases, gear motors, etc., composed for instance of a series of gears meshed with one another, fixed by means of shafts via friction bearings or bushes to what forms the surrounding housing of the mechanical component in question. This assembly is usually enclosed inferiorly in the so-termed crankcase, which acts as a reservoir containing the lubricating fluid that constitutes the reserve of lubricating fluid which will be projected over the moving mechanical components. As a result of the actual wear of all these mechanical components metal particles are detached, becoming incorporated into the actual lubricating fluid, and they are suitably filtered out before being projected over the mechanical components again.

With regard to these components, it is hard to ascertain their degree of wear as this would require laying up the vehicle where they are fitted and disassembling the whole unit in order to observe and carry out suitable corrective measures, wherefore preventive maintenance measures are adopted upon exceeding, for example, a number of operating hours which the manufacturer has stipulated for the servicing or repair of the machinery.

It is a known practice on vehicles and other types of machines to make use of either fixed or removable elements with magnetic characteristics, which, when inserted in the casing of the lubricating oil container reservoir, attract the possible ferromagnetic particles which are detached from the mechanical components, such as gears, bearings, etc., so that such particles may be withdrawn from the lubricating fluid, thereby preventing the filters from becoming prematurely unserviceable and such particles even being projected again towards the mechanical components, which would lead to excessive abrasion of these mechanical components.

Thus, for example, on motor vehicles, it is a classic solution to include this magnet in what is the lubricating fluid drain plug so that, when the lubricating oil is changed, this plug is cleaned and the particles adhered to it are therefore removed.

On certain machines, a filler and control cap is fitted that is provided with a rod with a magnetic material which attracts the particles detached as a result of that abnormal working and permits shut-downs to be utilized for carrying out schedules in which the oil level is checked and a diagnosis performed by the operators who carry out this supervision of oil levels to see if there is any potential anomaly that could be the cause of a serious problem in the future. This calls for the shut-down of the machine, the removal of this cap by the personnel, the checking and sometimes replacement of sealing washers or other associated spares, besides relying on the largely subjective judgment of the operator who carries out the intervention as to the potentiality of the problem or otherwise.

United States patent US-4,205,904 in favour of MASSEY-FERGUSON - SERVICES N.V. is already known. This refers to a device for detecting and indicating the presence of metal particles in a lubricating fluid, which is associated basically with a filtering device and, as such, when the metal particles clog this filter, electrical contact takes place between two electrodes that close an electrical circuit, which shows the need to proceed to replace or clean the filtering element. This device is made up of a ring connected to an electrical terminal fitted at one end of the filter, and a small chamber in series with the filtering device is created between the ring and the housing of the device. When the filter begins to be clogged with metal particles, that space also fills with such metal particles and electrical communication therefore takes place between both electrodes, which, being associated with an electrical circuit, will give a warning of the need for the filter to be changed.

This device is intended to forestall possible troubles in the mechanical components, but only for the purpose of assuring that the filtering elements are always in suitable condition for the through-flow of fluid and thereby assure an adequate supply of cooling liquid to those components.

United States patent US-5,402,113 of the firm AMOT CONTROLS CORPORATION refers to a metal particle detector device for non-conductor fluid systems. This detector is formed by means of two comb-like electrodes interlinked and separated from each other so as to achieve their insulation. These electrodes are supported on a perforated electrical insulating bearing strip, spaces being defined between these electrodes in which the metal particles are deposited and bring about the communication between both electrodes and the consequent closing of the circuit and, therefore, the signalling of the fact that the level of metal particles in the lubricating fluid has been exceeded.

This device is enclosed in a chamber through which the lubricating fluid is made to penetrate, but whose conditions do not make it possible to reflect the actual state of the machine where this device is deployed.

### DESCRIPTION OF THE INVENTION

The trend worldwide in the field of industrial maintenance is that of eradicating systematic routine preventive maintenance schedules which are carried out at constant intervals, in favour of more scientific maintenance schedules based on the evolution of a variable decisive for the state of the machine, which enable us to anticipate failures on the basis of data, analysis of said data or with alarm signals that will permit preventive measures to be scheduled in advance in accordance with each specific machine and each specific situation of the same.

Large expensive machines, as is the case of transmission cases, railway, windmill and other types of gear motors, torque multipliers, etc., obviously lie within the framework of reference of the trend that concerns us here.

Predictive diagnoses in these complex machines are based on vibration and oil analyses; these methods, which have shown their feasibility and efficacy in the last few years and which are implemented by a wide range of companies in different ways: continuously, by monitoring these variables all the time, by analysing their state taking advantage of other shut-downs, etc.

In any case, though very effective, these methods are economically expensive when we refer to a very extensive stock of machinery or when access to the same is complicated because of their awkward location, their being elements in operation and sometimes in movement, as happens with railway gear motors, etc.

This patent proposal sets out to offer a new system that provides an alarm situation prior to catastrophic failure by means of the inclusion of a simple sensoring system based on metal particles which are produced prior to catastrophic failure in any of these machines. In general terms, the basis is that of a known predictive technique called ferrography or analytical spectrometry, but simpler and much less expensive.

Any major problem in these machines, such as significant imbalances, lubrication failures, wear on the tooth of a gear, etc. is reflected in abnormal metal contact - metal which causes wear in the contact areas and detachment of metal chippings, usually of a ferrous origin. Unless the machine has elements stemming from copper, lead or other minerals, this kind of deterioration is potentially diagnosed by means of the technique of ferrography, which, based on the analysis of ferrous particles present in a sample of lubricant (quantitatively and qualitatively speaking) provides us with a diagnosis of the state and degree of progression of the deterioration.

The device advocated by the invention consists of a plug, preferably for screwing into the lubricant reservoir of the machinery whose wear is to be monitored, a plug which is prolonged in an elongated body that penetrates right into the lubricating fluid and will therefore be sized appropriately to extend between the wall of the container reservoir and the interior of the cooling fluid. This plug will form one of the poles of the electrical circuit, while the other pole will be formed of a bar magnet or magnetized rod which will be inserted concentrically into its interior, with an intermediate insulating piece fitted between the two in the form of a bushing whose outside dimensions will match the inner cavity of the plug and whose inside diameter will match the outside diameter of the bar magnet or magnetized material which will form the other pole, and both will be insulated by means of the intermediate insulating piece.

Accordingly, the end of the magnetized rod will be free and insulated from what is the body of the plug. When this device is inserted into the lubricating fluid, attraction of the metal particles takes place. These cling to the central bar magnet and the more the particles that are attracted, the greater their volume, until the time comes when the mass of particles is so high that they eventually come into contact with the actual body of the plug, which is when the circuit in which these electrodes lie is closed, indicating the excessive wear of the mechanical components to be monitored, so they ought to be changed or repaired.

To increase the contact surface between the two electrodes, i.e. between the bar magnet and the actual plug, slots or holes may be made in the side surface of the plug, similarly accompanied by slots or holes in the intermediate insulating piece, so that areas are achieved in which both are exposed and appropriately insulated by the intermediate insulating piece. This measure will assure greater effectiveness of the device through increasing the surface of action of the electrodes and therefore the increased capture of the ferrous metal particles that might be contained in the lubricating fluid, which will result in a longer duration of the filtering means and, therefore, in a highly exact and precise prediction of the state of the mechanical components of the machine in question.

### DESCRIPTION OF THE FIGURES

To supplement the description given and with a view to a better appreciation of the characteristics of the invention, in accordance with a preferred embodiment of the invention, as an integral part of the description, but not on a restrictive basis, we accompany it with the following figures:
Figure 1 represents a quarter-sectional view of a preferred form of plug according to the invention.
Figure 2 represents a sectional view of the insulating piece between the electrodes, which is inserted in the plug, as shown in Figure 1.
Figure 3 represents the bar magnet forming the electrode to which there cling the ferromagnetic particles stemming from the mechanical wear of the components to be monitored.
Figure 4 represents the device of the invention duly fitted.
Figure 5 represents the installation of the device of the invention in the crankcase of the machine that is intended to monitor.

### PREFERRED EMBODIMENT OF THE INVENTION

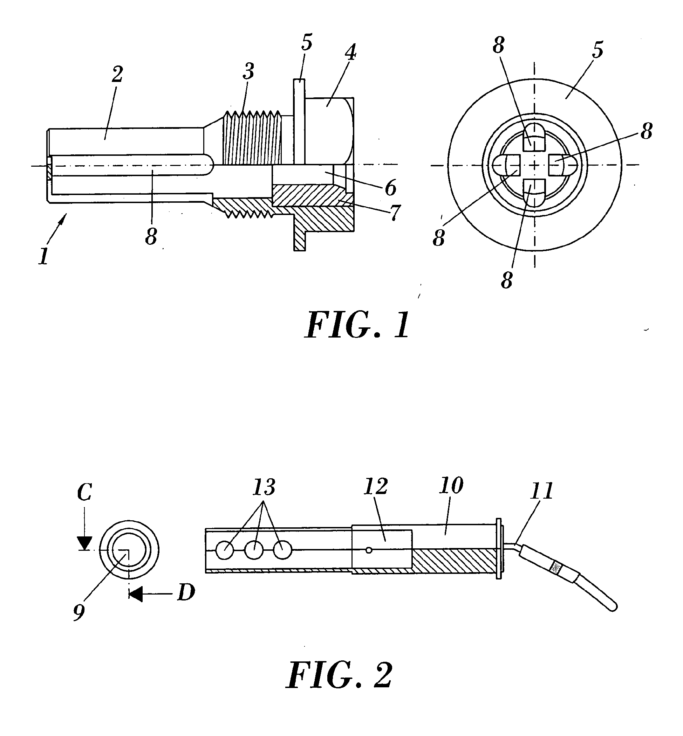

In Figure 1 we may observe a sectional view of the device of the invention, a device in the form of a plug (1) which is basically made up of three areas, the end area (2) which will come into contact with the lubricating fluid and will therefore interact with the magnet or magnetized electrode fitted inside the plug, a threaded area (3), which will be used as a connection to the body of the reservoir or crankcase where the above-mentioned plug is fastened, and an end area (4), which may be hexagonally shaped, for the tightening or tight fit of the aforesaid plug.

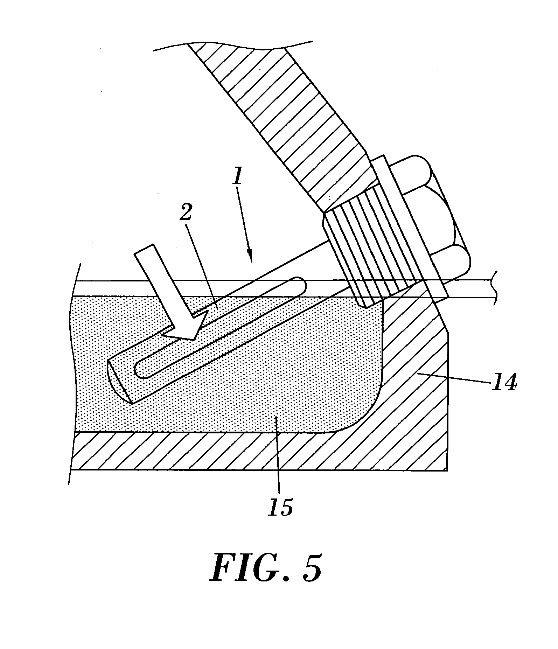

Between both areas (3) and (4), there is a ring (5) which acts as a stop in the penetration of the plug into the support bracket of the crankcase where the plug (1) is fitted, as may be observed in the adjoining Figure 5.

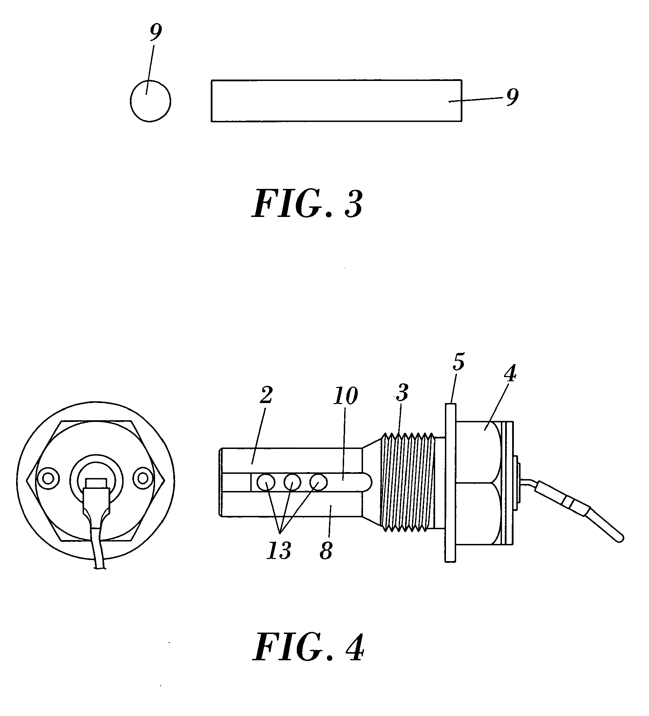

The interior of the plug (1) has a through-hole (6) in one of the ends of which a plastic adjusting bush (7) is interposed. Diametrically opposed, on the end area (2) there are preferably situated four slots (8) which would give access to the central electrode or bar magnet (9), as may be seen clearly in Figure 3.

Figure 2 represents the body (10) at the end of which there is a terminal (11) soldered forming the power supply for the electrode, whilst its interior is provided with a cavity (12) which houses the bar magnet electrode (9). The end area of this body (10) has holes (13), arranged in this case in four diametrically opposed rows aligned with the slots (8) in the body of the plug (1).

Figure 4 shows the installation of the device of the invention, in the representation of which we observe how access is obtained via the slots (8) to the body (10), appropriately provided with three holes (13) through which we observe the bar magnet (9) forming the body of the electrode.

Figure 5 shows the installation of the body of the plug (1) as it is inserted by way of the threaded hole in the crankcase (14), which holds a level of lubricating fluid (15) whose characteristics are intended to monitor. In this figure we may observe how the end of the plug (1), more specifically area (2), is immersed in the body of the lubricating fluid (15) so as to attract the ferromagnetic particles that might become detached from the mechanical components included in its interior.

## Claims

1. Detector for the predictive diagnosis of faults in medium and high power gear motors and transmission cases, **characterised in that** it consists of an enveloping electrode or hollow plug (1) in the interior of which there is coupled concentrically to it a second electrode (9) formed of a bar magnet or magnetized rod.

2. Detector for the predictive diagnosis of faults in medium and high power gear motors and transmission cases according to claim 1, **characterised in that** the body of the electrode or plug (1) is divided into different areas, an anterior one (2), through which the interior bar magnet or magnetized rod electrode (9) is visible and which is immersed in the lubricating fluid to be monitored, an intermediate threaded area (3) for connection to the reservoir or crankcase of the machine, and a third external area (4) for issuance of the electrode contacts, which includes the means for tightening the body of the plug on the reservoir or crankcase containing the lubricating fluid.

3. Detector for the predictive diagnosis of faults in medium and high power gear motors and transmission cases according to claim 1, **characterised in that** the enveloping electrode or plug (1) accommodates in its interior a plastic insulating bush (7) in whose inside diameter the bar magnet or magnetized rod (9) is fitted.

4. Detector for the predictive diagnosis of faults in medium and high power gear motors and transmission cases according to claim 1, **characterised in that** the end of the enveloping electrode or plug (1) which comes into contact with the lubricating fluid whose characteristics are to be monitored is provided with holes or slots (8) communicating with the hollow interior of the aforesaid plug (1) and therefore with the electrode (9).

5. Detector for the predictive diagnosis of faults in medium and high power gear motors and transmission cases according to claim 1 and 4, **characterised in that** it is provided with four diametrically-opposed holes or slots (8).

6. Detector for the predictive diagnosis of faults in medium and high power gear motors and transmission cases according to claim 1, **characterised in that** the bar magnet or magnetized rod electrode (9) is inserted in an enveloping retaining bush which includes holes or slots through which the bar magnet or magnetized rod electrode (9) is visible and acts.
